(19) Europäisches Patentamt

European Patent Office

Office européen des brevets

(11) Veröffentlichungsnummer: **0 264 374 B1**

(12) **EUROPÄISCHE PATENTSCHRIFT**

(45) Veröffentlichungstag der Patentschrift: **18.12.91**

(21) Anmeldenummer: **86904134.3**

(22) Anmeldetag: **19.06.86**

(86) Internationale Anmeldenummer:
**PCT/EP86/00361**

(87) Internationale Veröffentlichungsnummer:
**WO 86/07534 (31.12.86 86/28)**

(51) Int. Cl.⁵: **A61F 13/14, A61F 5/03**

(54) **SCHULTERBANDAGE.**

(30) Priorität: **20.06.85 CH 2606/85**

(43) Veröffentlichungstag der Anmeldung:
**27.04.88 Patentblatt 88/17**

(45) Bekanntmachung des Hinweises auf die
Patenterteilung:
**18.12.91 Patentblatt 91/51**

(84) Benannte Vertragsstaaten:
**BE CH DE FR GB IT LI NL SE**

(56) Entgegenhaltungen:
**FR-A- 758 255**
**FR-A- 1 361 918**
**FR-A- 2 368 263**
**US-A- 4 444 191**

(73) Patentinhaber: **TEMOVA ETABLISSEMENT**
**Landstrasse 140**
**FL-9494 Schaan(LI)**

(72) Erfinder: **Benckhuijsen, Gerrit Jan**
**Landstrasse 140**
**FL-9494 Schaan(LI)**

(74) Vertreter: **Büchel, Kurt F., Dr.**
**Bergstrasse 297**
**FL-9495 Triesen(LI)**

EP 0 264 374 B1

## Beschreibung

Die Erfindung bezieht sich auf eine Schulterbandage nach dem Oberbegriff des Anspruches 1.

Solche Bandagen wurden bisher vor allem bei Schlüsselbeinfrakturen eingesetzt und sind beispielsweise aus den US-A-3,856.004 und 3,857.388 bekannt. Sie zwingen zur Geradehaltung des Trägers, wobei seine Beweglichkeit extrem eingeschränkt ist, weil die Zugbänder nur wenig dehnbar sind, Rusksackträgern gleichen und meistens sehr nahe dem Halsbereich angeordnet sind.

Auch der Schultergürtel gemäss der US-A-3,548.818 schafft hier nur wenig Abhilfe, weil sowohl der flächige Rückenteil wie auch die Zugbänder im wesentlichen nur in Längsrichtung dehnbar sind und sich daher nur auf eine beschränkte Anzahl von Bewegungen, bzw. Freiheiten des Trägers auswirken.

Auch eine zu therapeutischen Zwecken einsetzbare elastische Weste mit Stützkragen ist beispielsweise aus der CH-A-641344 bekannt geworden. Dort geht es aber vor allem um die Abstützung im Cervikalbereich, bzw. um die Wärmewirkung im Schultergelenk.

Gegenüber den oberwähnten "statischen" Schlüsselbeinbandagen und auch dem erwähnten Schultergürtel geht die Erfindung von einer ganz anderen Problematik aus, der mit Hilfe einer allseitig "dynamischen" Schulterbandage begegnet werden soll. Bisher gab es nämlich keine Schulterbandage, die gerne getragen wurde, und die trotzdem ihre Wirkung nicht verfehlte.

Es hat nämlich der zivilisatorische Fortschritt zu einem hochentwickelten, vorwiegend sitzenden Menschen geführt, der sich daran gewöhnt hat, in einer krummen Stellung zu sitzen, durch die der Schultergütel, insbesondere das Acromio-Claviculargelenk, überbeansprucht wird. Damit wird die Wirbelsäule als tragende Stütze des Körpers nur noch teilweise ausgelastet, während andere Teile des Rumpfes, denen ursprünglich ganz andere Aufgaben zugedacht waren, wie z.B. das Brustbein, Funktionen als Stütz- und Halteapparate des Stammes übernehmen müssen. Da in entspannter Sitzstellung ("sternale Belastungshaltung") die Brustkyphose verstärkt und die Lendenlordose aufgehoben ist, bilden beide Wirbelsäulenabschnitte einen einheitlichen kyphotischen Bogen; die sternalen Gelenke werden - im Gegensatz zur Thoraxhaltung bei aufrechtem Stand und Gang - überbelastet.

Die axiale Belastung der Wirbelsäule ändert sich im Sitzen zu einer Beanspruchung auf Biegung; die Schwerkraftlinie verlagert sich nach ventral, und die Last von Kopf und Schultergürtel sowie Armen wird grösstenteils auf das Brustbein übertragen - dies bleibt nicht ohne Auswirkungen auf den Verlauf der Halswirbelsäule und auf die Gelenke des Schultergürtels, die in einen Reizzustand versetzt werden. Es kommt reflektorisch zu Verspannungen der Muskulatur und zu Schmerzsignalen zum Rückenmark und Hirnstamm. Dieser verarbeitet die Meldung über die Fehlbeanspruchung der Brustbeingelenke zu einer geordneten und wirksamen muskulären Abwehr, die der Entspannung der schmerzhaften Gelenke dienen soll. Die entsprechenden Muskeln werden innerviert (z.B. Muskulus sternocleidomastoideus, Muskuli scaleni, Rückenstrecker). Dies kann zu einer Ueberbeanspruchung dieser Muskulatur und demzufolge zu ausgebreiteten Schmerzen im gesamten Bereich der Brustwirbelsäule bis zum Hinterhaupt führen.

Bei der Behandlung hartnäckiger Cervicalsyndrome, Schulter-Arm-Syndrome und Thoracalsyndrome bedarf es einer Mitberücksichtigung dieser Mechanismen. Sie sind Folge bzw. sekundäre Begleiterscheinungen eines nozizeptiven somatomotorischen Blockierungseffektes, der seinen Ursprung in der Reizung der Schmerzsensoren (Nozizeptoren) der betroffenen Gelenkkapseln, Sehnenansätze, des Periostes und anderer Gewebe hat. Er ist Ausdruck reflektorischer Mechanismen, die dem Organismus zur Verfügung stehen, um die gereizten Gewebe zu schonen.

Aus der Erkenntnis dieser Zusammenhänge liegt der Erfindung die Aufgabe zugrunde, die Stützwirkung im Schulterbereich zu verbessern und der sternalen Belastungshaltung durch einen ständigen und dosierbaren Zug im Bereich des Schultergürtels entgegenzuwirken. Die nach eingehendem Studium des Haltungs- und Bewegungsapparates und seiner krankhaften Veränderungen sowie deren Auswirkungen in Form schmerzhafter Syndrome entwickelte erfindungsgemässe Schultergelenkbandage erreicht dies durch die im Kennzeichen des Anspruches 1 - vorzugsweise in Verbindung mit den in einem oder mehreren Unteransprüchen - genannten Merkmale.

Gegenüber der eingangs erwähnten Schlüsselbeinbandage wird durch die erfindungsgemässe Ausbildung eine Wirkung ganz anderer Art erreicht, und es hat sich gezeigt, dass dadurch nicht nur eine besondere Stützwirkung im Bereich des Schultergelenkes erzielbar ist, sondern sich auch ein sanfter Zwang zu einer geraden Haltung ergibt, ohne dass der Träger dabei in seinen Bewegungen behindert ist.

Der verbreiterte Pfannenteil am vorderen Zugbandende ist deswegen sehr wichtig, weil er die Oberarme in eine kleine Abspreizstellung bringt. Eine gewisse Bedeutung kommt auch der Auswahl des Abstandes der Zugbandbefestigung am Rückenteil von der Achselnaht zu. Die Kräfte müssen hier in geeigneter Weise in den Rückenteil einlaufen. Dies ist aber eine für den Fachmann von Fall zu Fall eruierbare Grösse.

Die beiden Zugbänder vereinigen ihre korrigierende Wirkung dorsal im Bereich des Scheitels der Brustkyphose. Die bei sternaler Belastungshaltung vor Brust- und Lendenwirbelsäule verlaufende Schwerkraftlinie wird normalisiert durch Verlagerung nach dorsal; die sternoclavikulären Gelenke, die Costosternalverbindungen und das Schultergelenk werden entlastet. Damit entfällt die mechanische Irritation von Bandapparat und Gelenken und der von hier ausgehende Reiz auf die Schmerzfühler dieser Region; der arthro-tendomyotische Reflexmechanismus wird unterbrochen.

Die bei sternaler Belastungshaltung überbeanspruchte Muskulatur (insbesondere M. sternocleidomastoideus, M. scaleni, Rückenstreckmuskulatur) kommt zur Entspannung, demgegenüber werden erwünschte Dehnungen des M. pectoralis minor und M. pectoralis major erreicht. Reizzustände mit dadurch bedingten Schmerzen in den Costosternalverbindungen sowie in den Clavicolosternalgelenken bilden sich zurück.

Es handelt sich deshalb um eine Bandage, die sinnvoll zur Behandlung von Schulterschmerzen, insbesondere zur Behandlung des Cervicalsyndroms und des Thoracalsyndroms, eingesetzt werden kann.

Die Verbreiterung der Zugbänder z.B. auf die Breite der Achselnaht ist deshalb von Vorteil, weil damit die Mahnfunktion der Bandage sanfter ausgeübt wird und daher die Bandage vom Patienten lieber getragen wird. Viele Heilmittel können nämlich oft ihre Wirkung nicht entfalten, weil sie von den Patienten entweder gar nicht oder in ungenügender Form angewendet werden. Darüber hinaus wird es durch die Verbreiterung der Zugbänder und unter Berücksichtigung der oben gegebenen Werte der Rückstellkräfte des Stoffes möglich, die Längsspannung über den Rücken in etwa der Längsspannung über die Brust anzugleichen. Bei schmäleren Zugbändern müsste zur Erreichung desselben Zieles ihre Spannung unangenehm hoch liegen.

Höhere als die angegebenen Rückstellkräfe, wie z.B. bei gewöhnlicher Maschenware in Längsrichtung, oder bei wenig oder gar nicht dehnfähigen Wickelbandagen, würden dazu führen, dass der Träger der Bandage in seinen Bewegungen beträchtlich gehindert ist. Das würde aber sowohl an sich die Heilung verlangsamen, als auch den Träger überhaupt die Bandage nur ungern und daher eher selten anlegen lassen.

Niedrigere als die angegebenen Rückstellkräfte, wie z.B. bei gewöhnlicher Maschenware in Querrichtung, oder auch bei hochelastischen Mieder- oder Schulterbekleidungen, führen dazu, dass der Träger auch bei für den Heilungsprozess ungünstiger Haltung zu wenig sanften Zug verspürt, als dass die Bandage noch als "Mahnbandage" bezeichnet werden könnte.

Bei der erfindungsgemässen Bandage ist es des weiteren bevorzugt, wenn das Zugband gegen die Körperaussenseite zu geschweift ausgebildet und am Rückenteil, vorzugsweise an der Seitennaht unter der Achsel, - gegebenenfalls verstellbar - befestigbar ist. Jedes Zugband überträgt nämlich Zugkräfte auf den Rückenteil, und es ist daher dafür zu sorgen, dass diese Kräfte optimal aufgenommen werden, ohne zu unangenehmer Faltenbildung zu führen. Dadurch, dass dabei das Zugband selbst die Pfanne bildet, werden die Kräfte nicht nur in Längsrichtung des Bandes weitergeleitet, sondern auch seitlich auf das Schultergelenk des Trägers übertragen, so dass sich nicht nur eine hohe Festigkeit ergibt, sondern zusätzlich der Stützeffekt im Bereiche des Schultergelenkes noch verbessert wird.

Wegen der aufzunehmenden Kräfte ist es günstig, wenn die Zugbänder aus verstärktem oder zweilagigem Stoff, insbesondere aus Maschenware, bestehen. Selbstverständ lich kann die Verstärkung auch durch eine entsprechende Materialwahl erfolgen. Maschenware ist deshalb besonders bevorzugt, weil sie bekanntlich eine gewisse Eigenelastizität besitzt. Gerade dann ist es aber vorteilhaft, wenn die Ränder der Zugbänder, vorzugsweise mit Hilfe von Säumbändern, eingesäumt sind, da ja einerseits Maschenware keine eigene Kante besitzt, anderseits die Möglichkeit der Saumbildung durch Verschweissung von Kunststoffmaterial eine Einbusse an Dehnbarkeit mit sich brächte. Die Säumbänder können dabei von Schrägbändern gebildet sein (d.i. gewebten Bändern mit schräg zur Längsachse gerichtetem Fadenverlauf) oder bestehen vorzugsweise auch aus Maschenware.

Für den Stützzweck genügt es an sich, wenn der Rückenteil einem etwa rechteckigen Streifen zwischen den Schultergelenken entspricht, doch mag er zur Erfüllung einer zusätzlichen Funktion gleich auch als Teil eines Kleidungsstückes und dabei hemd- oder leibchenartig ausgebildet sein.

Ansonsten ist es besonders günstig, wenn die Bandage mit einem Innen- und/oder Aussenhemd, gegebenenfalls lösbar, verbunden ist. Die Verbindung des Verstärkungsteiles mit dem hemdartigen Anlegeteil kann verhältnismässig lose sein (es ist auch möglich, überhaupt keine Verbindung vorzusehen), und sie ist zweckmässig lösbar.

Bei einer derartigen Ausbildung bewirkt das Innen- bzw. Unterhemd eine abpolsternde Unterlage, was insbesondere dann von Vorteil ist, wenn die Verbindung der Zugbänder im einfachsten Falle einfach durch Verknotung derselben nach Art von Schürzenbändern erfolgt. Das Aussen- oder Ueberhemd hingegen deckt die Bandage so ab, dass sie sich nicht oder nur wenig auf die Oberbekleidung durchdrückt.

Die gesamte Stützbekleidung erhält ein gefälliges Aussehen, wenn der hemdartige Anlegeteil aus zwei Lagen besteht, zwischen denen die erfindungsgemässe Schulterbandage angeordnet ist, wobei sie zumindest mit der darunter liegenden Innenlage zweckmässig durch kurze Nahtstellen am Anfang und Ende der Achselnaht und/oder der Seitennaht unter der Achsel verbunden ist. Die Schulterbandage ist dann auch gegen Verrutschen gut gesichert.

Bei dieser Abdeckung wird aber der Zugriff zu den hinter dem Rücken des Trägers zu befestigenden, bzw. zu verstellenden Zugbändern für eine Hilfsperson erleichtert, wenn die Aussenlage mindestens eine Oeffnung für den Durchgriff zu den am Rücken zu befestigenden Enden der Zugbänder aufweist. Diese Oeffnung kann von der am Rücken über ein Stück offen gebliebenen Bundnaht zwischen Innen- und Aussenhemd oder von Oeffnungen im Bereiche beider Seitennähte unter den Achseln, oder auch von gesonderten Schlitzöffnungen gebildet sein, etwa wenn der mit dem Verstärkungsteil verbundene Oberteil nach Art eines Trenchcoates über den Unterteil des Anlegeteiles geklappt ist und dazwischen einen Schlitz frei lässt.

Für den Stützzweck ist es bei gleichzeitiger Gewährleistung der vollen Beweglichkeit des Trägers besonders wichtig, wenn das Material der Stützbekleidung entsprechend den obien angegebenen Werten reversibel dehnbar ist, wobei beim Nachlassen der Zugspannung die Dehnung möglichst vollelastisch zurückgehen soll.

Beispiel:

Auf einer Rundstrickmaschine werden vier Spulen eines 40er Garnes (40.000 m Garn pro kg) aus 85% Polyvinylchlorid- und 15% Polyacrylnitrilfasern, vier Spulen eines umsponnenen Elastomergarnes zum Mitstricken und zwei Spulen desselben Elastomergarnes als Einlegefaden eingesetzt. Dieser Faden besteht aus 42 Gewichtsprozent Polyurethan-Elastomer (dtex 156) und 58 Gewichtsprozent Umspinnung aus texturiertem Polyamid 66 (dtex 44/13/1). Er weist eine Dehnung von 280% und - bei 0,5 g Vorlast - eine Lauflänge von 48.000 m pro kg auf. Es ergibt sich ein Gestrick mit beispielsweise 98% Rückstellung der Dehnung in Längsrichtung. Die Vorlast wird so gewählt, dass sich im Endprodukt nur 8,5% Elastomer befinden; die restlichen Bestandteile sind 62,3% Polyvinylchlorid, 11,0% Polyacrylnitril und 18,2% Polyamid. Das Material hat eine reversible Dehnung von 72% bei einer Belastung von 2,5 kg/100 mm.

Die Dehnung und ihre Rückstellfähigkeit in Querrichtung hängen von der Strickart ab. Ueblicherweise ergibt sich eine ausreichende Rückstellung erst jeweils nach dem Waschen, soferne dem Stoff nicht durch Mitlaufenlassen eines Gummifadens in der Querrichtung (auf Rundstrickmaschinen also quasi spiralig) eine entsprechende Dehnbarkeit und Rückstellfähigkeit gegeben wurde. Deshalb ist es bevorzugt, wenn der Rückenteil und/oder die Zugbänder in Längsrichtung der Strickware, jedoch quer über den Rücken gelegt werden. Das Material soll eine reversible Dehnung von mindestens 10, vorzugsweise von mindestens 40% aufweisen.

Als besonders vorteilhaft hat es sich auch erwiesen, wenn zwischen dem Zugband oder zumindest seinen Rändern einerseits und der Vorderseite des Schultergelenkes, auf die der Zug ausgeübt wird anderseits, eine Polsterung vorgesehen ist. Diese kann z.B. eine Lage eines gut atmungsaktiven Schaumstoffes sein, die entweder über die betreffende Länge des Zugbandes gelegt oder - da der Schaumstoff u.U. die elastische Dehnung des Zugbandes beeinträchtigt - besser am Innenhemd befestigt, z.B. mit Hilfe einer stoffgleichartigen Abdeckung eingenäht ist.

Weitere Einzelheiten ergeben sich an Hand der nachfolgenden Beschreibung von in der Zeichnung schematisch dargestellten Ausführungsbeispielen. Es zeigen:

Fig. 1       eine erste Ausführung der erfindungsgemässen Schulterbandage von vorne, nach dem Anlegen, jedoch vor dem Verbinden der beiden Zugbänder;

Fig. 1A      dieselbe Ausführung von der Rückenseite nach dem Verbinden der beiden Zugbänder;

Fig. 2       eine weitere Ausführungsform in einer der Fig. 1 ähnlichen Darstellung; und

Fig. 2A      diese Ausführungsform in einer der Fig. 1A entsprechenden Ansicht.

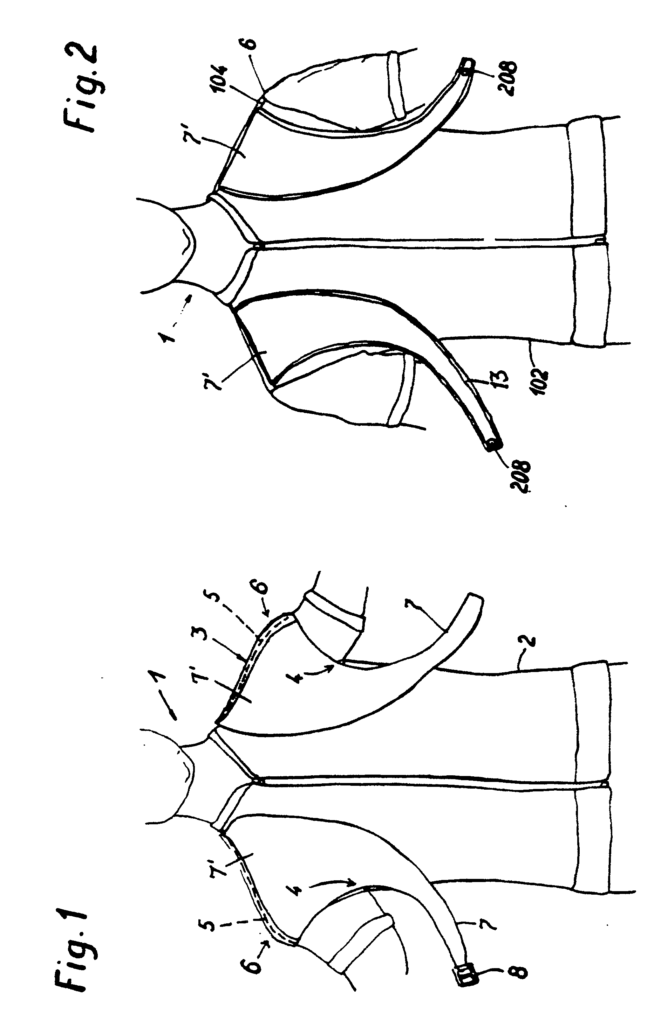

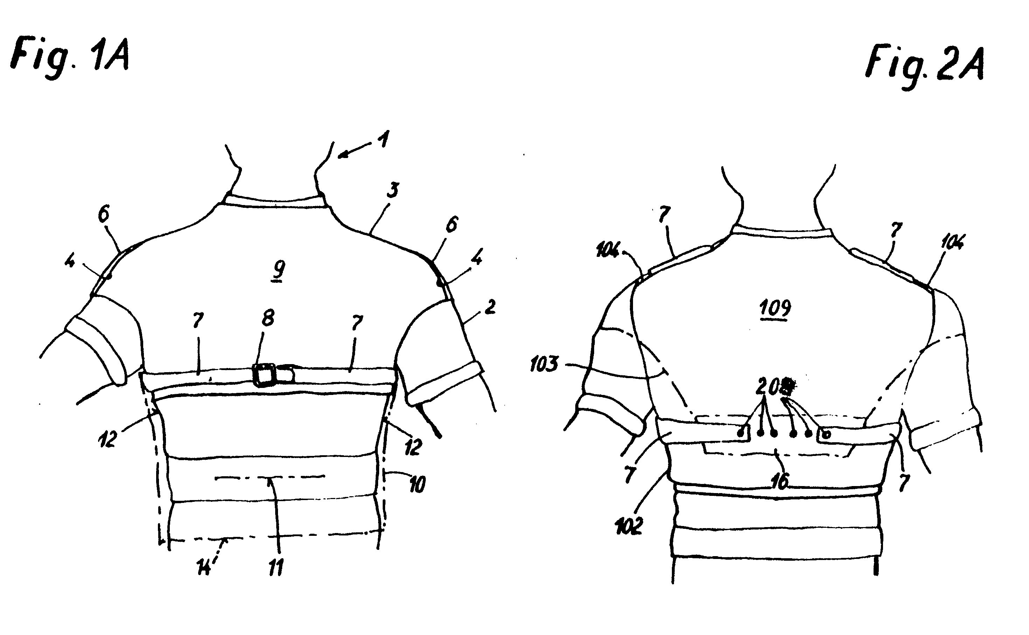

Eine therapeutisch zu behandelnde Person 1 hat gemäss Fig. 1 über einem Innenhemd 2 eine Schulterbandage 3 angelegt. Diese bildet durch eine gekrümmte Achselnaht 5 über den Achseln 4 der Person 1 je eine diese umhüllende Pfanne 6. Damit umgibt sie die Achsel 4 von mehreren Seiten. Die Elastizität der Schulterbandage 3 ergibt daher eine mehrseitige Stützwirkung im Bereich der Achsel 4.

Von der Achselnaht 5 geht je ein Zugband 7 aus, das in seinem oberen Bereich 7' relativ breit ausgebildet ist, um die Zugkräfte über einen grossen Abschnitt der Naht 5 aufzuteilen, während sich die Zugbänder gegen ihre freien Enden 7 zu verschmälern. Wie ersichtlich, sind die beiden Zugbänder 7 derart geschweift geschnitten, dass sie normalerweise mit ihren freien Enden gegen die Körperaussenseite weisen. Dadurch ist es ohne Faltenbildung möglich, sie unter der Achsel 4 des Trägers 1 hindurchzuführen und hinter dessen Rücken zu befestigen. Für diese Befestigung mag das Ende eines der Zugbänder mit einer Schnalle 8 versehen sein.

Das Innenhemd 2 kann gegebenenfalls Teil der Stützbekleidung sein, indem es mit der Schulterbandage 3 über die Achselnaht 5 verbunden ist. Um die Zugbänder 7 nicht sichtbar werden zu lassen, kann als Aussenlage noch ein ähnliches Hemd 10 darübergezogen sein, das gegebenenfalls mit dem Innenhemd 2 und/oder der Schulterbandage 3 verbunden ist. Diese Verbindung kann dann beispielsweise über einen Reissverschluss oder Druckknöpfe erfolgen, so dass das Aussenhemd getrennt waschbar ist. An sich wäre es möglich, Innenhemd und Aussenhemd mit der Schulterbandage 3 über die ganze Achselnaht 5 zu verbinden, doch ist es bevorzugt, wenn die Verbindung nur durch kurze Nahtstellen am Anfang und Ende der Achselnaht 5 und/oder der Seitennaht 12 unter der Achsel 4 erfolgt.

Im Gegensatz zu der schematischen Darstellung in der Zeichnung hat es sich übrigens als vorteilhaft erwiesen, wenn die Vorderseiten des Innen- und/oder Aussenhemdes (2,10) nicht hochgeschlossen, sondern stark ausgeschnitten sind, insbesondere für Damen, so dass die Brust nicht durch die doch immer noch verhältnismässig straff sitzenden Anlegeteile unter unangenehmen Druck gerät. Des weiteren mag es gerade bei Damen erwünscht sein, die Länge dieser Anlegeteile mit der Taille, also der engsten Stelle zu begrenzen, um einen guten Sitz sicherzustellen und das Hochrutschen zu vermeiden; für Herren hingegen mag eine längere Ausführung besser sein, die über den allfälligen Bauch reicht.

In Fig. 1A ist die Situation nach dem Verbinden der beiden Zugbänder 7 hinter dem Rücken des Trägers 1 gezeigt. Durch diese Verbindung ergibt sich ein Zug im Bereiche zwischen den Schulterblättern, der auf sanfte Weise für eine gerade Haltung des Trägers 1 sorgt. Es ist ersichtlich, dass der Rückenteil 9 der Schulterbandage 3 unterhalb der Pfannen 6 etwa rechteckförmig ist.

Falls auf das Innenhemd 2 verzichtet wird oder dieses von der eigentlichen Schulterbandage 3 getrennt ist, spielt der Rückenteil 9 mit seinem Anteil an den Pfannen auch die Rolle des Anlegeteiles, der einen festen Halt für die Zugbänder 7 gewährleistet. Ist jedoch die Schulterbandage 3 mit dem Innenhemd 2 verbunden, so dass dieses derjenige Teil ist, den der Träger 1 anlegt, so spielt der Rückenteil 9 mit den Pfannen 6 nur die Rolle eines Verstärkungsteiles, der den Zug der Zugbänder 7 aufzunehmen hat.

Wie schon erwähnt, kann die Schulterbandage 3 noch durch eine Aussenlage bzw. ein Aussenhemd 10 (strichpunktiert angedeutet) abgedeckt sein. In diesem Falle könnte eine die Verbindung der freien Enden der Zugbänder 7 über die Schnalle 8 herstellende oder verändernde Hilfsperson nur dann an die Schnalle 8 heran, wenn das Aussenhemd 10 hochgezogen wird. Zur Erleichterung der Handhabung ist deshalb vorzugsweise die Bundnaht 14 zwischen Innen- und Aussenhemd am Rücken über eine Strecke unterbrochen, oder im Aussenhemd ein Schlitz 11 vorzusehen.

Für die beiden Zugbänder 7 ist eine Verstärkung vorteilhaft, da sie ja die Spannkräfte aufzunehmen haben. Sie bestehen beispielsweise aus zwei Lagen, oder es wird durch die Materialwahl für eine Verstärkung gesorgt. Ausserdem ist aber eine hohe Elastizität mit einer Dehnbarkeit von 25% bis 60%, vorzugsweise von etwa 40%, besonders in Querrichtung des Rückenteiles 9, aber auch für die Zugbänder 7 vorteilhaft; daher wird nicht nur ein elastisches Fadenmaterial verwendet, wie z.B. Kräuselgarne aus PVC- und/oder Acrylfasern, die auch für eine günstige, die Durchblutung fördernde Reibungselektrizität sorgen; zweckmässig wird aus diesem Fadenmaterial ein Stoff in Form einer Maschenware erzeugt, die bekanntlich eine gewisse Eigenelastizität besitzt. Gerade hiefür ist es aber wichtig, einen Saum vorzusehen, der zweckmässig mit Hilfe elastischer Säumbänder 13 hergestellt wird.

Diese Säumbänder 13 umranden vorteilhaft die gesamte Schulterbandage 3. Dabei ist zu beachten, dass zwar jedes Zugband 7 aus zwei Lagen bestehen kann, dass dies aber im allgemeinen für den Rückenteil 9 nicht nur nicht erforderlich, sondern zur Vermeidung eines unangenehmen Hitzegefühles und des Schwitzens manchmal gar nicht zweckmässig ist. Der zweilagige oder verstärkte Stoff der Zugbänder endet also vorzugsweise an der Achselnaht 5.

Fig. 2 zeigt eine Ausführungsform, bei der der Rückenteil 9 selbst in das Innenhemd 102 eingearbeitet ist, an dessen Achselnaht 104 die Zugbänder 7 unmittelbar angenäht sind. Hier umgreift also nur das Innenhemd 102 selbst die Schultergelenke 6, und es ist ersichtlich, dass eine solche Ausführung zwar durch die dann erfolgende Verbindung der beiden Zugbandenden 208 hinter dem Rücken des Trägers 1 eine Straffung und Stützung der Wirbelsäule bewirkt; der Stützeffekt im Bereiche der Schultergelenkpfannen 6 des Trägers 1 bleibt aber natürlich geringer.

An Hand der Fig. 2A ist auch ersichtlich, dass die Verbindung der freien Enden der Zugbänder 7 nicht unbedingt unmittelbar miteinander erfolgen muss, sondern dass am Rückenteil 109 entsprechende Verbindungseinrichtungen 209 angeordnet werden können, hier in Form von Druckknöpfen oder Klettbändern, zu denen je ein Gegenstück an den Enden der Zugbänder 7 vorgesehen ist. Es kann dabei zur besseren Aufnahme der Kräfte vorteilhaft sein, in den Anlegeteil 102 zwischen den beiden freien Enden der Zugbänder 7 einen strichpunktiert angedeuteten Verstärkungsteil 16 einzuarbeiten.

Besonders bevorzugt ist es übrigens, wenn die Verbindungseinrichtungen 208 nicht am Rücken des Trägers 1 angebracht sind, wo sie auftragen und beim Anlehnen unangenehm empfunden werden, sondern an der Seitennaht 12 unter der Achsel 4. Besser als Druckknöpfe oder Klettbänder, die einem stärkeren Zug gegebenenfalls nicht gewachsen sind, ist auch die Verbindung von Häkchen und Oesen.

Im Rahmen der Erfindung ergeben sich verschiedene Variationsmöglichkeiten; beispielsweise kann es für rheumatische Schultergelenkserkrankungen zweckmässig sein, nicht nur eine Stützfunktion auszuüben, sondern auch besonders warmes Fasermaterial zu verwenden oder zumindest dem Material der Schulterbandage und/oder dem des Innen- und/oder Aussenhemdes beizumischen, wie etwa Angorawolle. Besonders vorteilhaft ist aber - zumindest für die der Haut des Trägers unmittelbar anliegenden Schichten - die Verwendung solcher Kunststoffasern, die auf der Haut Reibungselektrizität hervorrufen und dadurch die Durchblutung fördern.

## Patentansprüche

1. Schulterbandage, bestehend aus einem am Oberteil des Rückens angeordneten flächigen Rückenteil (9,109) und zwei Zugbändern (7), von denen je eines an jeder Seite an der Oberkante des Rückenteiles (9,109) ansetzt und über die Vorderseite des Schultergelenkes und unter der Achsel wieder auf den Rücken läuft und dort befestigbar ist, wobei der Rückenteil (9,109) und/oder die Zugbänder (7) aus elastischem Stoff ausgebildet sind, dadurch gekennzeichnet, dass die Rückstellkraft des Stoffes quer über den Rücken und längs über die Zugbänder (7) bei zehnprozentiger Dehnung gemessen an einem 5 cm breiten und 10 cm langen Streifen nach einem Vorschub von 5 cm pro Minute etwa 3.924 bis etwa 11.772 N, vorzugsweise 4.805 bis 7.848 N beträgt, und dass die Rückstellkraft des Stoffes längs über den Rücken und quer über die Zugbänder (7) bei zehnprozentiger Dehnung gemessen an 5 cm breiten und 10 cm langen Streifen nach einem Vorschub von 5 cm pro Minute etwa 1.962 bis etwa 4.905 N, vorzugsweise 2.943 bis 3.924 N beträgt.

2. Bandage nach Anspruch 1, dadurch gekennzeichnet, dass mit den Zugbändern (7) je ein an der lateralen Clavicula und in Höhe des Acromio-Claviculargelenkes angreifender Pfannenteil (6) verbunden ist.

3. Bandage nach Anspruch 2, dadurch gekennzeichnet, dass der Rückenteil (9,109) bis zur Achselnaht (5,104) reicht, wobei vorzugsweise das Zugband (7) in seinem Ansatzbereich (7') auf die Breite der Achselnaht (5,104) verbreitert ist.

4. Bandage nach Anspruch 3, dadurch gekennzeichnet, dass das Zugband (7) wenigstens zum Teil selbst einen Pfannenteil (6) bildet.

5. Bandage nach Anspruch 1, dadurch gekennzeichnet, dass das Zugband (7) gegen die Körperaussenseite zu geschweift ausgebildet und am Rückenteil (9,109), vorzugsweise an der Seitennaht (12) unter der Achsel (4), - falls verstellbar - befestigbar ist.

6. Bandage nach Anspruch 1, dadurch gekennzeichnet, dass die Zugbänder (7) aus verstärktem oder zweilagigem Stoff, insbesondere aus Maschenware, bestehen, wobei vorzugsweise ihre Ränder mit Hilfe von Säumbändern (13) eingesäumt und/oder von einem Polster, z.B. aus Schaumstoff, unterlegt sind.

7. Bandage nach Anspruch 1, dadurch gekennzeichnet, dass sie mit einem Innen- (2) und/oder Aussenhemd (10),gegebenenfalls lösbar, verbunden ist, wobei die Verbindung durch kurze Nahtstellen am Anfang und Ende der Achselnaht (5,104) und/oder der Seitennaht (12) unter der Achsel (4) erfolgt.

8. Bandage nach Anspruch 7, dadurch gekennzeichnet, dass die Bundnaht (14) zwischen Innen- (2) und Aussenhemd (10) am Rücken über eine Strecke unterbrochen, oder im Aussenhemd (10) ein Schlitz (11) vorgesehen ist (Fig. 1A).

9. Bandage nach einen der Ansprüche 1 bis 6, dadurch gekennzeichnet, dass der Rückenteil (109) Teil eines Hemdes, bzw. Leibchens (102) ist (Fig. 2A).

## Claims

1. Shoulder bandage consisting of a sheet-like back part (9, 109), arranged on the upper part of the back, and two pull bands (7), one of which is applied on each side to the upper edge of the back part (9, 109) and runs over the front of the shoulder and under the armpit to the back again and can be fastened there, the back part (9, 109) and/or the pull bands (7) being made of elastic material, characterized in that the restoring force of the material transversely over the back and longitudinally over the pull bands (7), measured at ten percent elongation on a 5 cm wide and 10 cm long strip after an advance of 5 cm per minute, is about 3.924 to about 11.772 N, preferably 4.805 to 7.848 N, and that the restoring force of the material longitudinally over the back and transversely over the pull bands (7), measured at ten percent elongation on 5 cm wide and 10 cm long strips after an advance of 5 cm per minute, is about 1.962 to about 4.905 N, preferably 2.943 to 3.924 N.

2. Bandage according to Claim 1, characterized in that a shoulder-conforming part (6) which covers the lateral clavicula and at the level of the acromioclavicular joint is connected to each of the pull bands (7).

3. Bandage according to Claim 2, characterized in that the back part (9, 109) extends as far as the shoulder seam (5, 104), the pull band (7) preferably being widened to the width of the shoulder seam (5, 104) in its region of attachment (7').

4. Bandage according to Claim 3, characterized in that the pull band (7) at least in part itself forms a shoulder-conforming part (6).

5. Bandage according to Claim 1, characterized in that the pull band (7) is tailored tapering away from the body and can be fastened - optionally adjustably - to the back part (9, 109), preferably to the side seam (12) under the shoulder (4).

6. Bandage according to Claim 1, characterized in that the pull bands (7) consist of reinforced or two-ply material, in particular of knitwear, their edges preferably being hemmed with trim bands (13) and/or being underlaid by a cushion, for example of foam.

7. Bandage according to Claim 1, characterized in that it is connected, optionally detachably, to an inside (2) and/or outside shirt (10), the connection being effected by short sections of seam at the beginning and end of the shoulder seam (5, 104) and/or the side seam (12) under the shoulder (4).

8. Bandage according to Claim 7, characterized in that the waist seam (14) between inside (2) and outside shirt (10) is interrupted over a distance at the back, or a slit (11) is provided in the outside shirt (10) (Fig. 1A).

9. Bandage according to any of Claims 1 to 6, characterized in that the back part (109) is part of a shirt or of a vest (102) (Fig. 2A).

## Revendications

1. Bandage scapulaire comprenant une partie dorsale aplatie (9, 109) disposée sur la région supérieure du dos, et deux bandes de traction (7) dont chacune se rattache de chaque côté à l'arête supérieure de la partie dorsale (9, 109), s'étend sur le côté antérieur de l'articulation scapulaire, et passe sous l'aisselle pour rejoindre le dos auquel elle peut être fixée, la partie dorsale (9, 109) et/ou les bandes de traction (7) étant réalisées en un matériau élastique, caractérisé par le fait que, mesurée pour un allongement de dix pour cent, la force de rappel du matériau, transversalement sur le dos et longitudinalement sur les bandes de traction (7), est d'environ 3,924 à environ 11,772 N, de préférence de 4,805 à 7,848 N sur une bande de 5 cm de largeur et de 10 cm de longueur, après une avance de 5 cm par minute ; et par le fait que, mesurée pour un allongement de dix pour cent, la force de rappel du matériau, longitudinalement sur le dos et transversalement sur les bandes de traction (7), est d'environ 1,962 à environ 4,905 N, de préférence de 2,943 à 3,924 N sur une bande de 5 cm de largeur et de 10 cm de longueur, après une avance de 5 cm par minute.

2. Bandage selon la revendication 1, caractérisé par le fait qu'une partie respective (6) formant cuvette, venant se placer sur la clavicule latérale et à la hauteur de l'articulation acromio-claviculaire, est reliée aux bandes de traction (7).

3. Bandage selon la revendication 2, caractérisé par le fait que la partie dorsale (9, 109) s'étend jusqu'au joint axillaire (5, 104), la bande de traction (7) étant de préférence élargie, dans sa zone de rattachement (7'), jusqu'à la largeur du joint axillaire (5, 104).

4. Bandage selon la revendication 3, caractérisé par le fait que la bande de traction (7) constitue elle-même, au moins partiellement, une partie (6) formant cuvette.

5. Bandage selon la revendication 1, caractérisé par le fait que la bande de traction (7) est réalisée échancrée en direction de la face externe du corps et peut être fixée à la partie dorsale (9, 109), de préférence au joint latéral (12) au-dessous de l'aisselle (4) - en cas de faculté de réglage -.

6.  Bandage selon la revendication 1, caractérisé par le fait que les bandes de traction (7) consistent en un matériau renforcé ou à deux couches, notamment en un tissu maillé, leurs bords étant de préférence ourlés à l'aide de lisières (13) et/ou garnis d'un rembourrage, par exemple en une mousse.

7.  Bandage selon la revendication 1, caractérisé par le fait qu'il est relié, éventuellement de manière libérable, à un maillot intérieur (2) et/ou extérieur (10), la solidarisation étant assurée par de courtes zones de jonction au début et à la fin du joint axillaire (5, 104) et/ou du joint latéral (12) au-dessous de l'aisselle (4).

8.  Bandage selon la revendication 7, caractérisé par le fait que le joint de liaison (14) entre les maillots intérieur (2) et extérieur (10) est interrompu, sur le dos, sur une certaine distance, ou bien une fente (11) est prévue dans le maillot extérieur (10) (figure 1A).

9.  Bandage selon l'une des revendications 1 à 6, caractérisé par le fait que la partie dorsale (109) fait respectivement partie d'un maillot ou d'un corset (102) (figure 2A).

*Fig.1*

*Fig.2*

EP 0 264 374 B1

Fig. 1A

Fig. 2A

EP 0 264 374 B1